# EUROPEAN PATENT APPLICATION

(11) **EP 4 566 605 A1**
(43) Date of publication of application: **11.06.2025**
(21) Application number: 23849520.4
(22) Date of filing: 04.08.2023
(51) Int. Cl.: A61K 31/4375, A61K 31/395, A61K 9/06, A61K 47/10, A61P 17/00, A61P 31/04

(54) **RIFAMYCIN-QUINOLIZINONE COUPLED MOLECULE OINTMENT AND METHOD FOR PREPARING SAME**

(30) Priority: 05.08.2022 CN 202210937430
(71) Applicant: Tennor Therapeutics (Zhongshan) Limited, Zhongshan, Guangdong 528451 (CN)
(72) Inventor: LIU, Yu, Suzhou, Jiangsu 215123 (CN); SONG, Ting, Suzhou, Jiangsu 215123 (CN); WANG, Huan, Suzhou, Jiangsu 215123 (CN); XU, Xiangyi, Suzhou, Jiangsu 215123 (CN); MA, Zhenkun, Suzhou, Jiangsu 215123 (CN)
(74) Representative: Weickmann & Weickmann PartmbB
(86) International application number: PCT/CN2023/111153
(87) International publication number: WO 2024/027816

(57) **Abstract**

The present application provides an ointment of a rifamycin-quinolizinone coupling molecule. The components of the ointment of rifamycin-quinolizinone coupling molecule comprise, based on the total mass percentage of 100%, 0.05%-0.7% by mass of the rifamycin-quinolizinone coupling molecule, 55%-65% by mass of polyethylene glycol 400, 25%-35% by mass of polyethylene glycol 3350, 8%-12% by mass of propylene glycol, 0.1%-1% by mass of a solubilizer, and 0.3%-1% by mass of an antioxidant. The ointment of rifamycin-quinolizinone coupling molecule can improve the drug loading capacity of the preparation and the stability of the medicament components, thereby improving the medication effect and user experience.

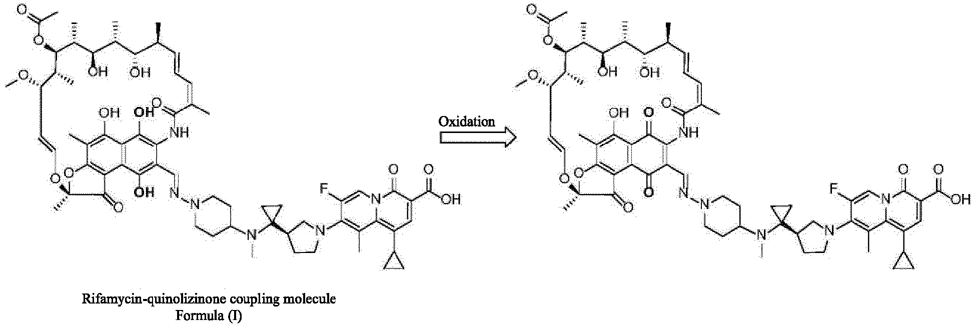

## Description

### TECHNICAL FIELD

The present application relates to an ointment of a rifamycin-quinolizinone coupling molecule and a preparation method therefor, and belongs to the technical field of medicine.

### BACKGROUND

The rifamycin-quinolizinone coupling molecule is a multi-target drug, the structural formula of which is shown as the following formula I:

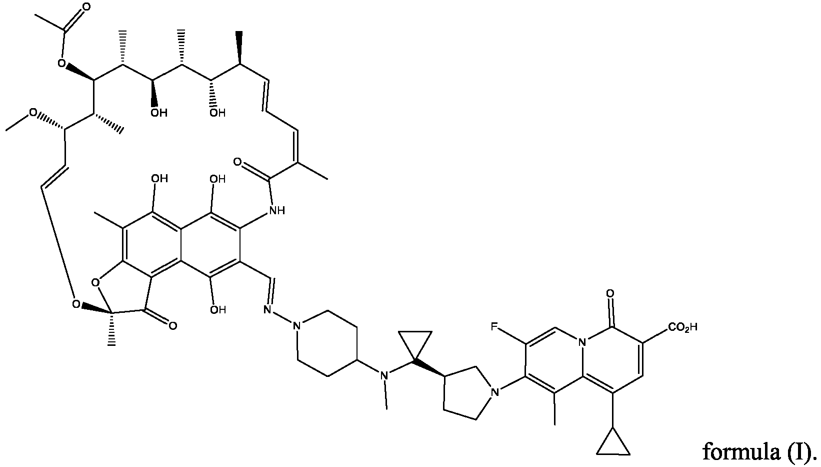

This molecule consists of rifamycin and quinolone pharmacophores and can effectively treat bacterial infections. The rifamycin-quinolizinone coupling molecule exhibits low water solubility, is soluble in dichloromethane, polyethylene glycol 400, and dimethyl sulfoxide, and is almost insoluble in ethanol, methanol, and diethyl ether. The water solubility of this molecule is pH-dependent, with maximum solubility observed at pH 10 and relatively low solubility at low pH values. In addition, the rifamycin-quinolizinone coupling molecule is easily to be oxidized when exposed to air, converting from its phenol form to its quinone form. At temperatures exceeding 60 °C, this molecule may be degraded. Under dry storage conditions, the primary degradation product is its quinone form. Oxidation or degradation of the compound may result in lower efficacy of the effective dose of the drug. Therefore, to broaden its application prospects, it is necessary to explore a series of improvements or development of certain specific formulations.

### SUMMARY

In view of the above defects existing in the prior art, the purpose of the present application is to provide an ointment of a rifamycin-quinolizinone coupling molecule, which can improve the drug loading capacity and the stability of drug components of the preparation, prevent the generation of oxidation products, improve the medication effect, and increase the medication safety.

The present application also aims to provide a preparation method for the ointment of rifamycin-quinolizinone coupling molecule, which can improve the drug loading and the stability of the product. The purposes of the present application are achieved by the following technical solutions:
In one aspect, the present application provides an ointment of a rifamycin-quinolizinone coupling molecule, wherein components of the ointment of the rifamycin-quinolizinone coupling molecule comprise: a rifamycin-quinolizinone coupling molecule as shown in formula I, polyethylene glycol 400, polyethylene glycol 3350, propylene glycol, a solubilizer, and an antioxidant; 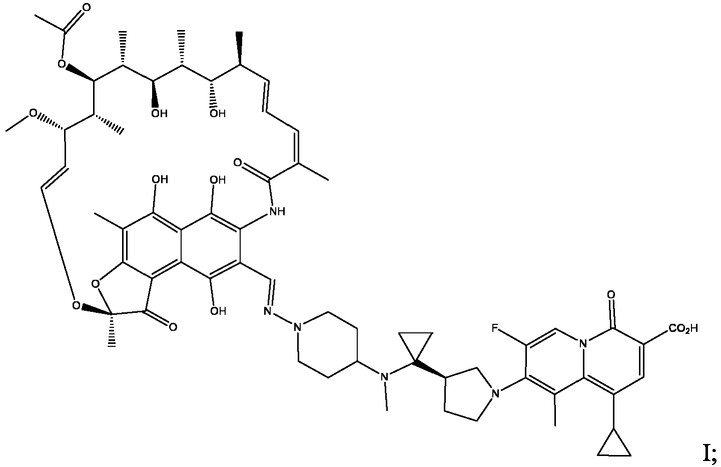 based on a total mass percentage of 100%, the content of the rifamycin-quinolizinone coupling molecule is 0.05%-0.7% by mass, the content of polyethylene glycol 400 is 55%-65% by mass, the content of polyethylene glycol 3350 is 25%-35% by mass, the content of propylene glycol is 8%-12% by mass, the content of the solubilizer is 0.1%-1% by mass, and the content of the antioxidant is 0.3%-1% by mass.

In some embodiments, the content of the rifamycin-quinolizinone coupling molecule is 0.3%-0.7% by mass.

In some embodiments, the content of the rifamycin-quinolizinone coupling molecule is 0.05%-0.5% by mass.

In some embodiments, the content of the rifamycin-quinolizinone coupling molecule is 0.5% by mass. In some embodiments, the content of polyethylene glycol 400 is 55%-60% by mass.

In some embodiments, the content of polyethylene glycol 400 is 56%-58% by mass.

In some embodiments, the content of polyethylene glycol 3350 is 28%-33% by mass.

In some embodiments, the content of polyethylene glycol 3350 is 30%-32% by mass.

In some embodiments, the content of the solubilizer is 0.3%-0.7% by mass.

In some embodiments, the solubilizer comprises triethanolamine.

In some embodiments, the content of the antioxidant is 0.5%-1% by mass.

In some embodiments, the antioxidant comprises one or more of vitamin C and vitamin E.

In some embodiments, the content of vitamin C and/or vitamin E is 0.3%-0.5% by mass.

In some embodiments, the components of the ointment of the rifamycin-quinolizinone coupling molecule comprise: a rifamycin-quinolizinone coupling molecule of formula I, polyethylene glycol 400, polyethylene glycol 3350, propylene glycol, a solubilizer, and an antioxidant; based on the total mass percentage of 100%, the content of the rifamycin-quinolizinone coupling molecule is 0.3%-0.7% by mass, the content of polyethylene glycol 400 is 55%-60% by mass, the content of polyethylene glycol 3350 is 28%-33% by mass, the content of propylene glycol is 8%-12% by mass, the content of the solubilizer is 0.1%-1% by mass, and the content of the antioxidant is 0.5%-1% by mass. In the above ointment of the rifamycin-quinolizinone coupling molecule, preferably, the solubilizer comprises triethanolamine. In the above ointment of the rifamycin-quinolizinone coupling molecule, preferably, the antioxidant comprises one or more of vitamin C and vitamin E.

In some embodiments, the components of the ointment of the rifamycin-quinolizinone coupling molecule comprise, based on the total mass percentage of 100%, 0.5% by mass of the rifamycin-quinolizinone coupling molecule, 56%-58% by mass of polyethylene glycol 400, 30%-32% by mass of polyethylene glycol 3350, 10% by mass of propylene glycol, 0.3%-0.7% by mass of triethanolamine, 0.3%-0.5% by mass of vitamin C, and 0.3%-0.5% by mass of vitamin E.

In another aspect, the present application provides a preparation method for the ointment of the rifamycin-quinolizinone coupling molecule described herein, which comprises: heating and melting polyethylene glycol 3350, propylene glycol, the solubilizer, the antioxidant, and part of polyethylene glycol 400, and mixing to obtain a first mixture; mixing and dissolving the rifamycin-quinolizinone coupling molecule and the rest of polyethylene glycol 400 to obtain a first drug liquid; and mixing the first drug liquid with the first mixture to obtain the ointment of the rifamycin-quinolizinone coupling molecule.

In some embodiments, in the step of mixing and dissolving the rifamycin-quinolizinone coupling molecule and the rest of polyethylene glycol 400 to obtain the first drug liquid, the mixing and dissolving are performed at a temperature of 50 °C.

In some embodiments, in the step of mixing the first drug liquid with the first mixture, the mixing is performed at a temperature of 50-53 °C.

In another aspect, the present application provides a preparation method for the ointment of the rifamycin-quinolizinone coupling molecule described herein, which comprises: heating and melting polyethylene glycol 3350, propylene glycol, the solubilizer, the antioxidant, and polyethylene glycol 400, and mixing to obtain a first mixture; and mixing the rifamycin-quinolizinone coupling molecule with the first mixture to obtain the ointment of the rifamycin-quinolizinone coupling molecule.

In some embodiments, the heating and melting are performed at a temperature of 70-73 °C.

In some embodiments, in the step of mixing the rifamycin-quinolizinone coupling molecule with the first mixture, the mixing is performed at a temperature of 50 °C.

### BRIEF DESCRIPTION OF THE DRAWINGS

The specific features of the invention to which the present application relates are set forth in the appended claims. The features and advantages of the invention to which the present application relates may be more fully understood with reference to the exemplary embodiments and drawings described in detail below. The drawings are briefly described as follows:
FIG. 1 shows the oxidative degradation reaction of the rifamycin-quinolizinone coupling molecule, resulting in the generation of an oxidation impurity.
FIG. 2 shows comparison results of electron microscopy of formulas with different solubilizers added.
FIG. 3 shows the bacterial load results in mice from Example 5 of the present disclosure.

### Detailed description

The technical solutions of the present disclosure are described in detail below to provide a clearer understanding of the technical features, objectives, and beneficial effects of the present disclosure. However, this description should not be constructed as limiting the embodiments of the present disclosure. In the following examples, the experimental methods are all conventional methods unless otherwise specified; the reagents and materials are commercially available, unless otherwise specified.

### Definitions of Terms

In the present application, the term "ointment" generally refers to a homogeneous semi-solid topical preparation obtained by mixing a drug with an oleaginous base or a water-soluble base. Due to the different dispersion states of the drug in the base, ointments may be classified into solution-type ointments and suspension-type ointments. The solution-type ointments are ointment dosage forms prepared by dissolving or co-dissolving the drug in the base or base components, while the suspension-type ointments are ointment dosage forms prepared by uniformly dispersing the fine drug powder in the base. The ointments provided by the present application are generally used in topical and local treatment, and in certain embodiments, the drug (e.g., the compound of formula I described herein) in the ointments can exert a systemic pharmacological or therapeutic effect upon transdermal absorption. In certain embodiments, the base of the ointment dosage form serves as an excipient and a pharmaceutical carrier, which can have an important influence on the quality of the ointment and the release and absorption of the drug. The bases of the ointment dosage forms commonly used in the art can be classified into oleaginous bases, emulsion bases, and water-soluble bases. The ointment dosage forms required for the treatment method of the present application may be obtained using suitable preparation methods and processes in the art.

In the present application, the term "melting" generally refers to a first-order phase transition process, which is accompanied by an increase in enthalpy, entropy, and volume, for example, as the temperature rises, the kinetic energy of the thermal motion of a molecule increases, leading to the disruption of the crystalline, and the transition process of a substance from a crystalline phase to a liquid phase may be referred to as melting. For example, in the present application, the process by which a substance that is solid at room temperature is heated to a certain temperature to reach its melting point and becomes a liquid substance, thereby acquiring certain physical properties of a liquid, may also be referred to as melting. The substance in the melted state may be in a liquid state or in a state of solid-liquid coexistence.

In the present application, the term "mixing" generally refers to a process of combining one or more compounds, cells, molecules, etc. in the same region. This process may be performed, for example, in a test tube, a culture dish, or any container that allows for the mixing of one or more compounds, cells, or molecules.

In the present application, all numbers disclosed herein are approximate values, whether or not the word "about" or "approximately" is used, and the numerical value of each number may vary, for example, by ±1%, ±2%, ±5%, etc. The term "about" or "approximately" is generally within an acceptable error range as determined by those of ordinary skills in the art for a particular value, which may depend in part on how the value is measured or determined, i.e., the limitations of the measurement system. For example, "about" or "approximately" may mean being within 1 or more than 1 standard deviation as per the practice in the art. Alternatively, "about" or "approximately" may mean a range of up to 10% or 20% (i.e., ±10% or ±20%). For example, about 3 mg may include any number between 2.7 mg and 3.3 mg (for 10%) or between 2.4 mg and 3.6 mg (for 20%). Furthermore, particularly for biological systems or processes, the term may mean a value up to an order of magnitude or up to 5 fold. Unless otherwise specified, when a particular value or composition is provided in this specification and the appended claims, the meaning of "about" or "approximately" should be assumed to be within an acceptable error range for the particular value or composition.

### Detailed Description of the Invention

In one aspect, the present application provides an ointment of a rifamycin-quinolizinone coupling molecule, wherein components of the ointment of the rifamycin-quinolizinone coupling molecule comprise: a compound rifamycin-quinolizinone coupling molecule of formula I, polyethylene glycol 400, polyethylene glycol 3350, propylene glycol, a solubilizer, and an antioxidant; based on a total mass percentage of 100%, the content of rifamycin-quinolizinone coupling molecule is 0.05%-0.7% by mass, the content of polyethylene glycol 400 is 55%-65% by mass, the content of polyethylene glycol 3350 is 25%-35% by mass, the content of propylene glycol is 8%-12% by mass, the content of the solubilizer is 0.1%-1% by mass, and the content of the antioxidant is 0.3%-1% by mass.

In another aspect, the present application provides a preparation method for the ointment of the rifamycin-quinolizinone coupling molecule described herein, which comprises: heating and melting polyethylene glycol 3350, propylene glycol, the solubilizer, the antioxidant, and part of polyethylene glycol 400, and mixing to obtain a first mixture; mixing and dissolving the rifamycin-quinolizinone coupling molecule and the rest of polyethylene glycol 400 to obtain a first drug liquid; and mixing the first drug liquid with the first mixture to obtain the ointment of the rifamycin-quinolizinone coupling molecule.

In another aspect, the present application provides a preparation method for the ointment of the rifamycin-quinolizinone coupling molecule described herein, which comprises: heating and melting polyethylene glycol 3350, propylene glycol, the solubilizer, the antioxidant, and polyethylene glycol 400, and mixing to obtain a first mixture; and mixing the rifamycin-quinolizinone coupling molecule with the first mixture to obtain the ointment of the rifamycin-quinolizinone coupling molecule.

### Rifamycin-quinolizinone coupling molecule

The rifamycin-quinolizinone coupling molecule (API) is a multi-target compound, which consists of rifamycin and quinolone pharmacophores and has a structural formula shown as formula I, 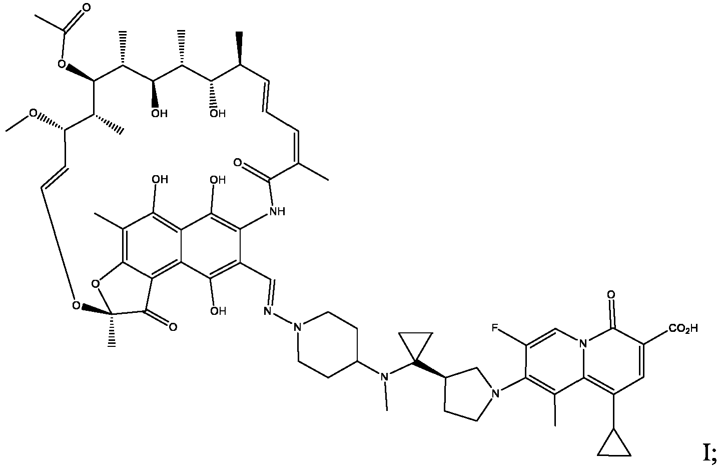 this molecule has antibacterial activity against bacteria such as *Staphylococcus*, *Streptococcus*, and *Streptococcus pneumoniae*, and its topical formulations can be used for treating skin infections. The drug acts on three different targets, including RNA polymerase, DNA gyrase, and topoisomerase IV. This multi-target mechanism of action can achieve the technical effect of reducing the possibility of drug resistance. The rifamycin-quinolizinone coupling molecule is prepared from TenNor Therapeutics.

In some embodiments, the content of the rifamycin-quinolizinone coupling molecule is 0.05%-0.7% by mass. For example, the rifamycin-quinolizinone coupling molecule may be present in an amount of about 0.05%, about 0.06%, about 0.07%, about 0.08%, about 0.09%, about 0.1%, about 0.15%, about 0.2%, about 0.25%, about 0.3%, about 0.35%, about 0.4%, about 0.45%, about 0.5%, about 0.55%, about 0.6%, about 0.65%, or about 0.7% of the total mass.

In some embodiments, the content of the rifamycin-quinolizinone coupling molecule is 0.3%-0.7% by mass. For example, the rifamycin-quinolizinone coupling molecule may be present in an amount of about 0.3%, about 0.35%, about 0.4%, about 0.45%, about 0.5%, about 0.55%, about 0.6%, about 0.65%, or about 0.7% of the total mass.

In some embodiments, the content of the rifamycin-quinolizinone coupling molecule is 0.05%-0.5% by mass. For example, the rifamycin-quinolizinone coupling molecule may be present in an amount of about 0.05%, about 0.06%, about 0.07%, about 0.08%, about 0.09%, about 0.1%, about 0.15%, about 0.2%, about 0.25%, about 0.3%, about 0.35%, about 0.4%, about 0.45%, or about 0.5% of the total mass.

In some embodiments, the content of the rifamycin-quinolizinone coupling molecule is 0.5% by mass.

### Polyethylene glycol 400 and polyethylene glycol 3350

The solubility of the rifamycin-quinolizinone coupling molecule is relatively low, and in order to obtain an ointment with a suitable drug loading capacity and stability, the application provides an ointment base comprising a combination of polyethylene glycol 400 and polyethylene glycol 3350.

In some embodiments, the content of polyethylene glycol 400 is 55%-65% by mass. For example, polyethylene glycol 400 may be present in an amount of about 55%, about 56%, about 57%, about 58%, about 59%, about 60%, about 61%, about 62%, about 63%, about 64%, or about 65% of the total mass.

In some embodiments, the content of polyethylene glycol 400 is 55%-60% by mass. For example, polyethylene glycol 400 may be present in an amount of about 55%, about 56%, about 57%, about 58%, about 59%, or about 60% of the total mass.

In some embodiments, the content of polyethylene glycol 400 is 56%-58% by mass. For example, polyethylene glycol 400 may be present in an amount of about 56%, about 56.1%, about 56.2%, about 56.3, about 56.4, about 56.5, about 56.6%, about 56.7%, about 56.8%, about 56.9%, about 57%, about 57.1%, about 57.2%, about 57.3, about 57.4, about 57.5, about 57.6%, about 57.7%, about 57.8%, about 57.9%, or about 58% of the total mass.

In some embodiments, the content of polyethylene glycol 3350 is 25%-35% by mass. For example, the content of polyethylene glycol 3350 may be present in an amount of about 25%, about 26%, about 27%, about 28%, about 29%, about 30%, about 31%, about 32%, about 33%, about 34%, or about 35% of the total mass.

In some embodiments, the content of polyethylene glycol 3350 is 28%-33% by mass. For example, the content of polyethylene glycol 3350 may be present in an amount of about 28%, about 29%, about 30%, about 31%, about 32%, about 33%, about 34%, or about 35% of the total mass.

In some embodiments, the content of polyethylene glycol 3350 is 30%-32% by mass. For example, polyethylene glycol 3350 may be present in an amount of about 30%, about 30.1%, about 30.2%, about 30.3, about 30.4, about 30.5, about 30.6%, about 30.7%, about 30.8%, about 30.9%, about 31%, about 31.1%, about 31.2%, about 31.3, about 31.4, about 31.5, about 31.6%, about 31.7%, about 31.8%, about 31.9%, or about 32% of the total mass.

### Propylene glycol

The ointment of the present application not only considers the stability of the formula, but also improves the relatively hard spreading property caused by the polyethylene glycol bases by adding a certain proportion of propylene glycol, thereby caring the use experience of patients or users as well. In some embodiments, the content of propylene glycol is 8%-12% by mass. For example, propylene glycol may be present in an amount of about 8%, about 8.5%, about 9%, about 9.5%, about 10%, about 10.5%, about 11%, about 11.5%, or about 12% of the total mass. Specifically, the content of propylene glycol may be present in an amount of about 10% of the total mass.

### Solubilizer

To address the low solubility of the rifamycin-quinolizinone coupling molecule and its possible adverse tendency of API precipitation or aggregation, an appropriate solubilizer is also added to the ointment components of the present application. In some embodiments, the content of the solubilizer is 0.1%-1% by mass. For example, the solubilizer may be present in an amount of about 0.1%, about 0.15%, about 0.2%, about 0.25%, about 0.3%, about 0.35%, about 0.4%, about 0.45%, about 0.5%, about 0.55%, about 0.6%, about 0.65%, about 0.7%, about 0.75%, about 0.8%, about 0.85%, about 0.9%, about 0.95%, or about 1% of the total mass.

In some embodiments, the content of the solubilizer is 0.3%-0.7% by mass. For example, the content of the solubilizer may be present in an amount of about 0.3%, about 0.31%, about 0.32%, about 0.33%, about 0.34%, about 0.35%, about 0.36%, about 0.37%, about 0.38%, about 0.39%, about 0.4%, about 0.41%, about 0.42%, about 0.43%, about 0.44%, about 0.45%, about 0.46%, about 0.47%, about 0.48%, about 0.49%, about 0.5%, about 0.51%, about 0.52%, about 0.53%, about 0.54%, about 0.55%, about 0.56%, about 0.57%, about 0.58%, about 0.59%, about 0.6%, about 0.61%, about 0.62%, about 0.63%, about 0.64%, about 0.65%, about 0.66%, about 0.67%, about 0.68%, about 0.69%, or about 0.7% of the total mass.

In some embodiments, the content of the solubilizer may comprise triethanolamine. For example, the ointment of the rifamycin-quinolizinone coupling molecule described herein may comprise triethanolamine in an amount about 0.3%, about 0.31%, about 0.32%, about 0.33%, about 0.34%, about 0.35%, about 0.36%, about 0.37%, about 0.38%, about 0.39%, about 0.4%, about 0.41%, about 0.42%, about 0.43%, about 0.44%, about 0.45%, about 0.46%, about 0.47%, about 0.48%, about 0.49%, about 0.5%, about 0.51%, about 0.52%, about 0.53%, about 0.54%, about 0.55%, about 0.56%, about 0.57%, about 0.58%, about 0.59%, about 0.6%, about 0.61%, about 0.62%, about 0.63%, about 0.64%, about 0.65%, about 0.66%, about 0.67%, about 0.68%, about 0.69%, or about 0.7% of the total mass.

### Antioxidant

The stability during storage and the oxidative degradation of the ointment of the rifamycin-quinolizinone coupling molecule described herein may be improved by adding an antioxidant thereto. In some embodiments, the content of the antioxidant is 0.3%-1% by mass. For example, the antioxidant may be present in an amount of about 0.3%, about 0.35%, about 0.4%, about 0.45%, about 0.5%, about 0.55%, about 0.6%, about 0.65%, about 0.7%, about 0.75%, about 0.8%, about 0.85%, about 0.9%, about 0.95%, or about 1% of the total mass.

In some embodiments, the content of the antioxidant is 0.5%-1% by mass. For example, the antioxidant may be present in an amount of about 0.5%, about 0.51%, about 0.52%, about 0.53%, about 0.54%, about 0.55%, about 0.56%, about 0.57%, about 0.58%, about 0.59%, about 0.6%, about 0.61%, about 0.62%, about 0.63%, about 0.64%, about 0.65%, about 0.66%, about 0.67%, about 0.68%, about 0.69%, about 0.7%, about 0.71%, about 0.72%, about 0.73%, about 0.74%, about 0.75%, about 0.76%, about 0.77%, about 0.78%, about 0.79%, about 0.8%, about 0.81%, about 0.82%, about 0.83%, about 0.84%, about 0.85%, about 0.86%, about 0.87%, about 0.88%, about 0.89%, about 0.9%, about 0.91%, about 0.92%, about 0.93%, about 0.94%, about 0.95%, about 0.96%, about 0.97%, about 0.98%, about 0.99%, or about 1% of the total mass.

In some embodiments, the antioxidant comprises one or more of vitamin C and vitamin E. For example, the antioxidant may be vitamin C, vitamin E, or a combination of vitamin C and vitamin E. For example, the ointment of the rifamycin-quinolizinone coupling molecule described herein may comprise vitamin C, vitamin E, or a combination of vitamin C and vitamin E in an amount of about 0.5%, about 0.51%, about 0.52%, about 0.53%, about 0.54%, about 0.55%, about 0.56%, about 0.57%, about 0.58%, about 0.59%, about 0.6%, about 0.61%, about 0.62%, about 0.63%, about 0.64%, about 0.65%, about 0.66%, about 0.67%, about 0.68%, about 0.69%, about 0.7%, about 0.71%, about 0.72%, about 0.73%, about 0.74%, about 0.75%, about 0.76%, about 0.77%, about 0.78%, about 0.79%, about 0.8%, about 0.81%, about 0.82%, about 0.83%, about 0.84%, about 0.85%, about 0.86%, about 0.87%, about 0.88%, about 0.89%, about 0.9%, about 0.91%, about 0.92%, about 0.93%, about 0.94%, about 0.95%, about 0.96%, about 0.97%, about 0.98%, about 0.99%, or about 1% of the total mass.

In some embodiments, the content of vitamin C and/or vitamin E is 0.3%-0.5% by mass. For example, vitamin C may be present in an amount of about 0.3%, about 0.31%, about 0.32%, about 0.33%, about 0.34%, about 0.35%, about 0.36%, about 0.37%, about 0.38%, about 0.39%, about 0.4%, about 0.41%, about 0.42%, about 0.43%, about 0.44%, about 0.45%, about 0.46%, about 0.47%, about 0.48%, about 0.49%, or about 0.5% of the total mass, and/or vitamin E may be present in an amount of about 0.3%, about 0.31%, about 0.32%, about 0.33%, about 0.34%, about 0.35%, about 0.36%, about 0.37%, about 0.38%, about 0.39%, about 0.4%, about 0.41%, about 0.42%, about 0.43%, about 0.44%, about 0.45%, about 0.46%, about 0.47%, about 0.48%, about 0.49%, or about 0.5% of the total mass.

### Preparation method for ointment

The preparation method for the ointment of the rifamycin-quinolizinone coupling molecule described herein comprises heating and melting polyethylene glycol 3350, propylene glycol, the solubilizer, the antioxidant, and polyethylene glycol 400 to obtain a first mixture, wherein part of quantitative polyethylene glycol 400 may be added, or all of quantitative polyethylene glycol 400 may be added during the heating and melting. In some cases, the heating and melting are performed at a temperature of not lower than 70 °C. For example, the heating and melting may be performed at a temperature of about 70 °C, about 71 °C, about 72 °C, about 73 °C or higher. The preparation method described herein further comprises mixing the rifamycin-quinolizinone coupling molecule with the first mixture containing all of polyethylene glycol 400, and in some cases, the mixing step is performed at a temperature of not lower than 50 °C, for example, the mixing step may be performed at a temperature of about 50 °C, about 51 °C, about 52 °C, about 53 °C or higher; or the preparation method described herein further comprises mixing and dissolving the rifamycin-quinolizinone coupling molecule and the rest of polyethylene glycol 400 from the step of heating and melting to obtain the first mixture, and then mixing the resulting solution with the first mixture, wherein the mixing and dissolving, as well as the mixing with the first mixture, are all performed at a temperature of not lower than 50 °C, for example, the mixing and dissolving, as well as the mixing with the first mixture, may be performed at a temperature of about 50 °C, about 51 °C, about 52 °C, about 53 °C or higher.

Without being bound by any theory, the following examples are intended only to illustrate the ointment, the preparation method therefor, the use thereof, etc., of the present application, and are not intended to limit the scope of the present application.

### Examples

### Example 1

### Selection of Ointment Bases

An ointment, as a relatively safe topical preparation, is convenient to use and has targeted action on infections of the skin and skin tissues, making it a preferable choice. Commonly used ointment bases include PEG400 (i.e., polyethylene glycol 400), etc. Considering the relatively low solubility of the rifamycin-quinolizinone coupling molecule, it is necessary to select the base to ensure relatively good solubility of the drug in the base, thereby enhancing the drug loading capacity of the preparation.

Experimental steps: About 340 mg of API was added gradually and slowly to about 5 g of PEG400 (in a water bath at 40 °C), and the mixture was dissolved by magnetic stirring. The orange-red mixture was gradually viscous and could no longer be stirred. About 7 g of PEG400 was added gradually and stirred for another 20 min. The resulting mixture was divided into two portions, one portion was maintained in a water bath at 40 °C, and the other portion was cooled to room temperature. Both portions were filtered through a PES water membrane (13 mm, 0.45 µm), and the filtrates were collected for content analysis.

About 400 mg of API was added gradually and slowly to about 5 g of propylene glycol (in a water bath at 40 °C), and the mixture was stirred for dissolution. The orange-red mixture was gradually viscous and could no longer be stirred. About 7 g of PEG400 was added gradually and stirred for another 20 min. The resulting mixture was divided into two portions, one portion was maintained in a water bath at 40 °C, and the other portion was cooled to room temperature. Both portions were filtered through a PES water membrane (13 mm, 0.45 µm), and the filtrates were collected for content analysis.

The 4 filtrates were tested for the drug content, as shown in Table 1 below.

**Table 1. Solubility of API in bases**

| Base + API | Test temperature | Saturation solubility (mg/g) |
|---|---|---|
| PEG400+API | Room temperature | 5.80 |
| | 40 °C | 23.79 |
| Propylene glycol + API | Room temperature | 0.15 |
| | 40 °C | 0.65 |

As shown in the above table, the solubility of the rifamycin-quinolizinone coupling molecule in PEG400 at 40 °C exceeds the formulation specification (specification: 0.5%, i.e., 5 mg/g), which is superior to the solubility at room temperature and significantly better than other bases, such as propylene glycol. Therefore, PEG400 was selected as the primary base.

However, when PEG400 was used as the sole base for an ointment, the ointment was relatively hard, with poor spreading property. After multiple experiments, different proportions of propylene glycol were added to the base to obtain the following formulas for comparative testing. The formula composition is shown in Table 2 below:

**Table 2. Investigation of different base formulas**

| Test batch No. | Formula 1 | Formula 2 | Formula 3 | Formula 4 | Formula 5 | Formula 6 | Formula 7 |
|---|---|---|---|---|---|---|---|
| Formula composition | Proportion (%) | Proportion (%) | Proportion (%) | Proportion (%) | Proportion (%) | Proportion (%) | Proportion (%) |
| PEG400 | 65 | 70 | 80 | 55 | 58 | 60 | 65 |
| PEG3350 | 35 | 30 | 20 | 35 | 32 | 30 | 25 |
| Propylene glycol | NA | NA | NA | 10 | 10 | 10 | 10 |

Experimental steps: The excipients were weighed out according to the formula amounts and processed using a one-pot method. All excipients from each batch were added simultaneously in one portion and heated in a water bath at 65-73 °C with magnetic stirring. The mixture was slowly stirred before melting. After melting, the mixture was stirred at 1500 rpm for 10 min, and then stirred at 800 rpm at room temperature and cooled to solidify.

The base formulas of the above different proportions were left to stand in a refrigerator at 2-8 °C overnight. The next day, the formulas were taken out and allowed to return to room temperature. The appearance and microscopic images of the products were observed, and the spreading properties were evaluated. Through comparison, it was observed that formula 1, formula 2, and formula 3 exhibited a wax-like appearance, and particularly, formula 3 showed waxy solid-liquid separation; formula 4, formula 5, formula 6, and formula 7 exhibited a snowflake-like structure and became gradually thinner. The spreading properties of the corresponding 7 formulas transitioned from being spreadable to being easily and uniformly spreadable. The microscopic observation showed that the bases in formula 4 and formula 5 exhibited relatively more uniform distribution, making them more suitable as bases.

It can be seen from the tests that the advantages of propylene glycol used include: 1. propylene glycol has a lower viscosity compared to PEG400, is miscible with PEG400, can be melted and mixed with PEG3350, and can significantly improve the sensory properties of polyethylene glycol hydrophilic ointments, which are relatively hard; 2. propylene glycol, as a commonly used humectant in topical formulations, can maintain the consistency of the ointment; and 3. propylene glycol has certain antibacterial activity. In the example of the present application, the comparison was performed on the bases of ointments, and the products in the example of the present application can solve the problems that the existing ointments exhibit a relatively hard waxy texture and have an unsatisfactory spreading property.

### Example 2

### Selection of Oxidants and Amounts Thereof

This example provides an ointment of the rifamycin-quinolizinone coupling molecule, and the components of the ointment of the rifamycin-quinolizinone coupling molecule comprise, based on the mass percentage, the rifamycin-quinolizinone coupling molecule in an amount of 0.5% of the total mass, polyethylene glycol 400 in an amount of 56%-58% of the total mass, polyethylene glycol 3350 in an amount of 30%-32% of the total mass, propylene glycol in an amount of 10% of the total mass, vitamin C in an amount of 0-0.5% of the total mass, vitamin E in an amount of 0-0.5% of the total mass, and BHT in an amount of 0-0.5% of the total mass.

The ointment of the rifamycin-quinolizinone coupling molecule of this example was prepared by the following method:
Polyethylene glycol 3350, propylene glycol, an antioxidant, and polyethylene glycol 400 were added to a container, and the mixture was heated and melted in a water bath at 70-73 °C, and slowly and mechanically stirred at a rotation speed of 1000 rmp until a clear solution was obtained; the mixture was stirred for another 10 min and mixed to obtain a first mixture; the rifamycin-quinolizinone coupling molecule was mixed with the first mixture in a water bath at 50-53 °C, and the mixture was mechanically stirred at 1000 rpm for about 15 min until no rifamycin-quinolizinone coupling molecule powder was observed by naked eyes; and the mixture was continuously stirred and cooled to 40-43 °C to obtain the ointment of the rifamycin-quinolizinone coupling molecule.

The ointments of the rifamycin-quinolizinone coupling molecule of formulas 8-12 were all prepared using this method, except that the formula composition of each excipient was slightly different.

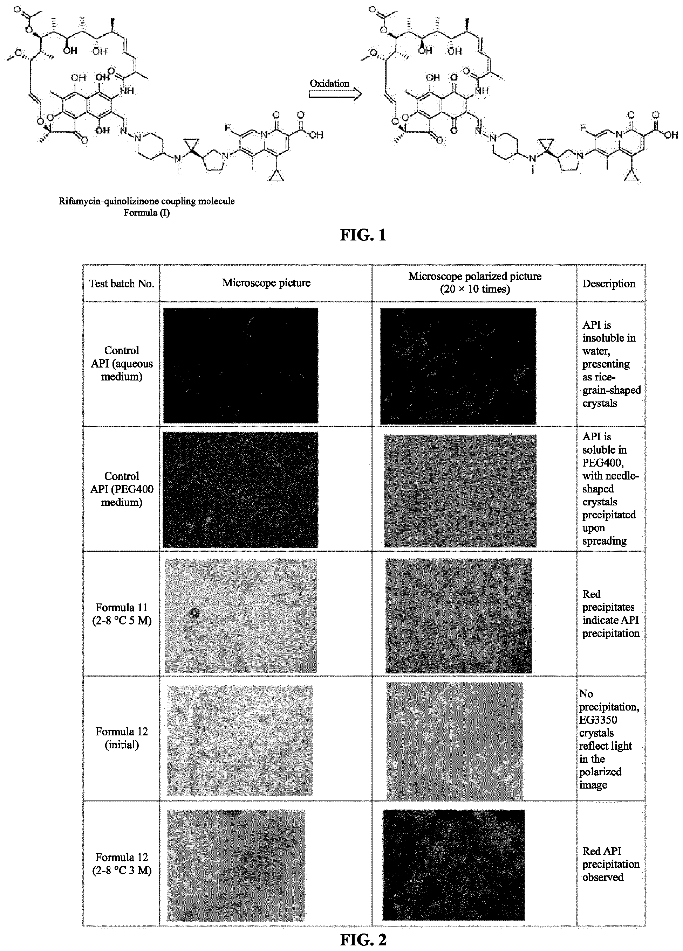

During the preparation process, the rifamycin-quinolizinone coupling molecule may undergo oxidative degradation when exposed to air, resulting in the generation of an oxidation impurity, and the structural change resulting from the reaction is shown in FIG. 1. To reduce the oxidative degradation rate of the rifamycin-quinolizinone coupling molecule, the single use or combined use of several antioxidants such as butylated hydroxytoluene (BHT), vitamin C, and vitamin E, as well as the usage amounts thereof, were investigated. The formulas are shown in Table 3.

**Table 3. Investigation of different antioxidant formulas**

| Test batch No. | Formula 8 | Formula 9 | Formula 10 | Formula 11 | Formula 12 |
|---|---|---|---|---|---|
| Formula composition | Proportion (%) | Proportion (%) | Proportion (%) | Proportion (%) | Proportion (%) |
| Rifamycin-quinolizinone coupling molecule | 0.5 | 0.5 | 0.5 | 0.5 | 0.5 |
| PEG400 | 57.6 | 58.0 | 57.6 | 56.0 | 57.4 |
| PEG3350 | 31.8 | 32.0 | 31.4 | 30.0 | 31.3 |
| Propylene glycol | 10.0 | 10.0 | 10.0 | 10.0 | 10.0 |
| Vitamin C | NA | 0.5 | NA | 0.5 | 0.3 |
| Vitamin E | NA | NA | 0.5 | 0.3 | 0.5 |
| BHT | 0.1 | NA | NA | NA | NA |

According to the formula composition of the above table, the ointments of the rifamycin-quinolizinone coupling molecule were prepared, and the related substances were investigated. The results are shown in Table 4 below:

**Table 4. Results of antioxidant investigation on related substances**

| Test batch No. | Formula 8 | | | Formula 9 | | | Formula 10 | | | Formula 11 | | | Formula 12 | | |
|---|---|---|---|---|---|---|---|---|---|---|---|---|---|---|---|
| Antioxidant | BHT | | | 0.5% vitamin C | | | 0.5% vitamin E | | | 0.5% vitamin C + 0.5% vitamin E | | | 0.3% vitamin C + 0.5% vitamin E | | |
| Investigation condition | 0 day | Room temperature for 7 days | 0 day | 5 °C/3 M | 30 °C/3 M | 0 day | 5 °C/3 M | 30 °C/3 M | 0 day | 5 °C/3 M | 5 °C/5 M | 30 °C/3 M | 0 day | 5 °C/3 M | 30 °C/1 M |
| Oxidation impurity | 0.37 | 3.55 | 0.16 | NA | 0.09 | 0.76 | 1.59 | 0.28 | 0.14 | NA | 0.1 | 0.06 | 0.02 | NA | NA |
| Total impurities | 0.99 | 5.45 | 0.93 | 2.18 | 10.44 | 1.58 | 5.64 | 79.04 | 0.81 | 1.69 | 2.11 | 6.68 | 0.64 | 1.55 | 4.62 |

As shown in the above table, after formula 8 was left to stand at room temperature for 7 days, the oxidation impurity increased from 0.37% to 3.55%, and the total impurities also increased significantly, indicating that BHT failed to reduce the generation of the oxidation impurity through degradation. However, the stability of the formula could be significantly improved by adding the antioxidant VC (VC is short for vitamin C), and the oxidative degradation was significantly inhibited. Among these formulas, formula 11 exhibited the smallest increase in both oxidation impurity and total impurities, indicating that the antioxidant combination of 0.5% VC + 0.5% VE (VE is short for vitamin E) has a better effect on improving the stability of the formula. In the example of the present application, the comparison was performed on the types and proportions of the antioxidants, and the products of the example of the present application can improve the problems that the existing ointments may undergo degradation to generate oxidation impurities and have low formula stability.

### Example 3

### Screening and Investigation of Solubilizers

The ointments prepared in Example 2 was subjected to microscopic examination for comparison, and the results are shown in FIG. 2. The material of formula 11 had a tendency of red API precipitation or aggregation after being stored at 2-8 °C for 5 months. Although the content uniformity was acceptable, RSD was relatively high (5.5%), which may be attributed to API precipitation, resulting in unsatisfactory content uniformity values.

Triethanolamine was added as a solubilizer in the example of the present disclosure based on the unsatisfactory solubility of the API itself. One carboxylic acid group and three phenolic hydroxyl groups in the structure of the rifamycin-quinolizinone coupling molecule can provide H⁺. Triethanolamine provides a lone pair of electrons and can form an ion-pair complex with the organic acid group of the rifamycin-quinolizinone coupling molecule. The ion-pair complex has lower lattice energy, and thus the rifamycin-quinolizinone coupling molecule is more easily dissolved in solvent. Meanwhile, triethanolamine exhibits as a base, and is preliminarily used to adjust the pH of ointment to be neutral. The acid-base property of the ointment could be investigated. The addition amount of triethanolamine was preliminarily calculated from the addition amounts of API and vitamin C. Theoretically, triethanolamine provides a lone pair of electrons, is a Lewis base, and can form a salt with carboxylic acid or a Lewis acid at room temperature without catalysis. The reaction formula is N-(CH₂CH₂OH)₃+HCOOH = HCOO⁻+[HN-(CH₂CH₂OH)₃]⁺. If 1 mol of vitamin C in the ointment provides 1 mol of H⁺, one carboxylic acid group and three phenolic hydroxyl groups in the API structure can provide H⁺. Taking 100 g of ointment as an example, 0.4235 g of triethanolamine was needed for 0.5 g of vitamin C in the neutralization formula, and 0.26 g of triethanolamine was needed for neutralizing H⁺ provided by one carboxylic acid group and three phenolic hydroxyl groups in API. Thus, the addition amount of triethanolamine should be theoretically less than 0.4235 g + 0.26 g = 0.6835 g. Based on this calculation, a gradient of 0.3%, 0.5%, and 0.7% of the triethanolamine formula addition amount was set.

Based on formula 11, 0.3%, 0.5%, and 0.7% of triethanolamine were added to obtain formulas 13-15, respectively, and the effects of different formulas on the pH, related substances, and properties of ointments were investigated.

**Table 5. Investigation of formula pH**

| Test batch No. | Formula 11 | Formula 13 | Formula 14 | Formula 15 |
|---|---|---|---|---|
| Formula composition | Proportion (%) | Proportion (%) | Proportion (%) | Proportion (%) |
| Rifamycin-quinolizinone coupling molecule | 0.5 | 0.5 | 0.5 | 0.5 |
| PEG 400 | 56.0 | 57.1 | 57.0 | 56.9 |
| PEG 3350 | 30.0 | 31.1 | 31.0 | 30.9 |
| Propylene glycol | 10.0 | 10.0 | 10.0 | 10.0 |
| VC | 0.5 | 0.5 | 0.5 | 0.5 |
| VE | 0.3 | 0.5 | 0.5 | 0.5 |
| Triethanolamine | NA | 0.3 | 0.5 | 0.7 |
| Average pH value (measured after two-fold dilution with pure water) | 4.4 | 5.8 | 7.0 | 7.5 |
| Average pH value (measured directly) | 5.5 | 6.8 | 7.1 | 7.2 |

| | | | | |
|---|---|---|---|---|
| Note: 1. When diluted with pure water, the sample was manually shaken until it was visually homogeneous. 2. The directly measured pH were obtained from 3 measurements of the same batch of samples; the pH (measured after two-fold dilution with pure water) were obtained from 3 measurements of the sample diluted two-fold in the same centrifuge tube. | | | | |

As shown in the above table, the addition of triethanolamine could adjust the pH of the ointment from weakly acidic to neutral.

**Table 6. Stability results of formulas with different addition amounts of triethanolamine**

| Related substance | | 0 day | 5 °C | | | 30 °C | |
|---|---|---|---|---|---|---|---|
| | | | 10 days | 30 days | 3M | 10 days | 30 days |
| Test batch No. | Impurities | Impurities (%) | Impurities (%) | Impurities (%) | Impurities (%) | Impurities (%) | Impurities (%) |
| Formula 11 | Acid degradation impurities | NA | 0.08 | 0.25 | 0.6 | 0.96 | 2.1 |
| | Total impurities | 0.81 | 0.77 | 1.15 | 1.69 | 1.71 | 3.53 |
| Formula 13 | Acid degradation impurities | NA | 0.04 | 0.06 | 0.27 | 0.63 | 1.8 |
| | Total impurities | 0.49 | 0.68 | 0.65 | 1.32 | 1.67 | 3.8 |
| Formula 14 | Acid degradation impurities | NA | 0.04 | 0.07 | 0.21 | 0.5 | 1.59 |
| | Total impurities | 0.5 | 0.73 | 0.84 | 1.12 | 1.79 | 3.83 |
| Formula 15 | Acid degradation impurities | NA | 0.04 | 0.05 | 0.12 | 0.34 | 1.11 |
| | Total impurities | 0.54 | 0.73 | 0.73 | 1.05 | 1.75 | 3.4 |

The structure of acid degradation impurity was shown as the formula below:

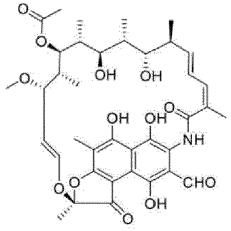

It can be seen from the stability results of the batches that when the addition amounts of triethanolamine were 0.3%, 0.5%, and 0.7%, the total impurities of the samples after 3 months of storage at 2-8 °C were 1.32%, 1.12%, and 1.02%, respectively, indicating an improvement in chemical stability compared to formula 11 only an antioxidant added, where the amount of total impurities was 1.69% (Table 7). Furthermore, as can be seen from Table 6, no API precipitation or aggregation was observed in 3 batches stored at 5 °C for 3 months, indicating the improved physical stability and demonstrating the solubilization effect.

### Example 4

### Preparation of the Ointment of Rifamycin-quinolizinone coupling Molecule

This example provides an ointment of a rifamycin-quinolizinone coupling molecule, and the components of the ointment of the rifamycin-quinolizinone coupling molecule comprise, based on the mass percentage, the rifamycin-quinolizinone coupling molecule in an amount of 0.5% of the total mass, polyethylene glycol 400 in an amount of 56% of the total mass, polyethylene glycol 3350 in an amount of 32% of the total mass, propylene glycol in an amount of 10% of the total mass, triethanolamine in an amount of 0.5% of the total mass, vitamin C in an amount of 0.5% of the total mass, and vitamin E in an amount of 0.5% of the total mass.

The ointment of the rifamycin-quinolizinone coupling molecule of this example was prepared by the following method:
Polyethylene glycol 3350 (PEG3350 for short), propylene glycol, a solubilizer, an antioxidant, and polyethylene glycol 400 (PEG400 for short) were added to a container, and the mixture was heated and melted in a water bath at 70-73 °C, and slowly and mechanically stirred at a rotation speed of 1000 rmp until a clear solution was obtained; the mixture was stirred for another 10 min and mixed to obtain a mixture;
the rifamycin-quinolizinone coupling molecule was mixed with the mixture in a water bath at 50-53 °C, and the mixture was mechanically stirred at 1000 rpm for about 15 min until no rifamycin-quinolizinone coupling molecule powder was observed by naked eyes; and the mixture was continuously stirred and cooled to 40-43 °C to obtain the ointments of the rifamycin-quinolizinone coupling molecule.

The ointments of the rifamycin-quinolizinone coupling molecule were all prepared using this method, except that the formula composition of each excipient was slightly different.

### Example 5

### Antibacterial Activity Test of Ointments of Rifamycin-quinolizinone Coupling Molecule on Skin Surface

*Staphylococcus aureus* (*S. aureus*) is one of the most common bacterial pathogens in human and can cause skin and soft tissue infections (SSTIs), bacteremia, osteomyelitis, and other disseminated infections. Penicillin-binding protein PBP2a is the main cause of drug resistance in *S. aureus.* PBP2a has a low binding capacity to β-lactam antibiotics, and can maintain the stability of bacterial peptidoglycan in the presence of such antibiotics, thereby conferring drug resistance to the bacteria. Strains containing PBP2a are referred to as methicillin-resistant *Staphylococcus aureus* (MRSA). *S*. *aureus* NRS 384 is one such strain, notable in the United States for causing infections in healthcare settings, and primarily associated with skin and soft tissue infections. The ointment of the rifamycin-quinolizinone coupling molecule, prepared by TenNor Therapeutics (Suzhou) Limited, is an ointment preparation intended for the treatment of *S*. *aureus* infections. In this experiment, a mouse skin infection model was established using *S. aureus* NRS 384 to evaluate the antibacterial activity of the ointments of the rifamycin-quinolizinone coupling molecule on the skin surface.

Experiment design: in this experiment, 6 groups were set, including a blank ointment group, a Bactroban ointment group, and three dose groups (low-, medium-, and high-dose groups) of ointments of rifamycin-quinolizinone coupling molecule. Each group contained 8 animals, and the specific grouping is shown in Table 7.

**Table 7. Administration regimen**

| **Group** | **Number of animals** | **Inoculation dose** | **Treatment regimen Transdermal spreading** | **Administration time** | **Endpoint of experiment** | **Detection Indicator** |
|---|---|---|---|---|---|---|
| Blank ointment | 8 | *S.aureus* NRS 384 5.0E+07 CFU/mouse | NA | 4 h; 19 h; 26 h; 43 h; 50 h; 67 h | 72 h | CFU in skin (bacterial load) |
| Bactroban ointment | 8 | | 30 mg/mouse (containing 0.6 mg of mupirocin) | | | |
| ointment with 0.5% rifamycin-quinolizinone coupling molecule | 8 | | 30 mg/mouse (containing 0.15 mg of rifamycin-quinolizinone coupling molecule) | | | |
| ointment with 0.05% rifamycin-quinolizinone coupling molecule | 8 | | 30 mg/mouse (containing 0.015 mg of rifamycin-quinolizinone coupling molecule) | | | |
| ointment with 0.005% rifamycin-quinolizinone coupling molecule | 8 | | 30 mg/mouse (containing 0.0015 mg of rifamycin-quinolizinone coupling molecule) | | | |

### Experimental results:

### 1) Counting results of inoculated bacterial solution:

The CFU counting results of the inoculated bacterial solution were as follows: the actual concentration of the inoculation solution was 3.85E+09 CFU/mL, and the inoculation amount of each mouse was 3.85E+07 CFU.

**Table 8. Inoculation dose**

| **Strain** | **Dilution factor** | **Spot plate volume (µL)** | **Number of CFUs** | **Average value** | **CFU/mL** | **Inoculation volume (µL)** | **Inoculation route** | **Actual inoculation amount (CFU/mouse)** |
|---|---|---|---|---|---|---|---|---|
| S.aureus NRS 384 | 10⁶ | 10 | 37/40 | 38.5 | 3.85E+09 | 10 | Transdermal spreading | 3.85E+07 |

### 2) Counting results of bacterial load on mouse skin:

**Table 9. Counting results of bacterial load on skin**

| **Group** | **Strain** | **Treatment regimen Transdermal spreading, b.i.d.** | **Administration time** | **Bacterial load on skin Lg (CFU/mouse)** |
|---|---|---|---|---|
| Blank ointment | *S.aureus* NRS 384 3.85E+07 CFU/mouse | Blank ointment for 72 h | 4 h; 19 h; 26 h; 43 h; 50 h; 67 h | 9.154±0.273 |
| Bactroban ointment | | 2% Bactroban ointment 30 mg/mouse | | 7.035±0.591^{*} |
| ointment containing 0.5% rifamycin-quinolizinone coupling molecule | | ointment containing 0.5% rifamycin-quinolizinone coupling molecule 30 mg/mouse | | 4.436±0.850^{***} |
| ointment containing 0.05% rifamycin-quinolizinone coupling molecule | | ointment containing 0.05% rifamycin-quinolizinone coupling molecule 30 mg/mouse | | 6.054±1.060^{***} |
| ointment containing 0.005% rifamycin-quinolizinone coupling molecule | | ointment containing 0.005% rifamycin-quinolizinone coupling molecule 30 mg/mouse | | 8.091±0.687 |

| | | | | |
|---|---|---|---|---|
| Note: Compared to the model group, p*** < 0.001, indicating highly significant differences; compared to the model group, 0.01 < p* < 0.05, indicating significant differences; a one-way ANOVA analysis method was used. | | | | |

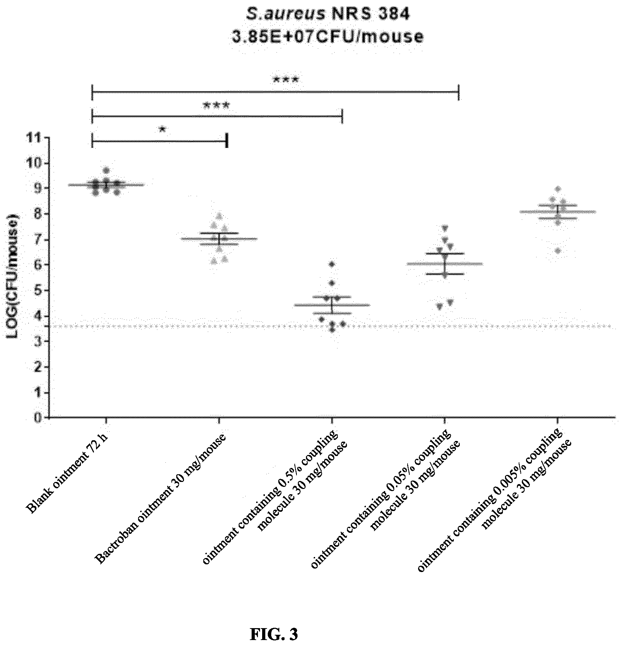

The results of this pharmacodynamic experiment showed that the bacterial load on the skin of the blank ointment group was 9.154±0.273 1g. A stable skin infection model could be constructed using *S. aureus* NRS 384 (Table 9, FIG. 3). Compared to the blank ointment group, the bacterial load on the mouse skin was reduced by 2.119 Log after the treatment of 30 mg/mouse of the Bactroban ointment (2% mupirocin), indicating the Bactroban ointment has a good bactericidal effect; the animals treated with 0.5%, 0.05%, and 0.005% ointments of the rifamycin-quinolizinone coupling molecule, respectively, showed reductions in the bacterial load on the skin of 4.718 Log, 3.100 Log, and 1.063 Log, indicating that the ointments of the rifamycin-quinolizinone coupling molecule in the high-dose groups of 0.5% and 0.05% exhibited good bactericidal effects in this skin infection model, which had highly significant differences from the animals in the blank ointment group.

### Experimental conclusion:

In this study, a stable mouse skin infection model was constructed using *S. aureus* NRS 384 to evaluate the antibacterial activity of the ointments of the rifamycin-quinolizinone coupling molecule on the skin surface. The results show that the ointment of the rifamycin-quinolizinone coupling molecule can effectively reduce the bacterial load on the mouse skin and shows dose-dependent bactericidal activity.

## Claims

1. An ointment of a rifamycin-quinolizinone coupling molecule, wherein the components of the ointment of rifamycin-quinolizinone coupling molecule comprise:
rifamycin-quinolizinone coupling molecule of formula I, polyethylene glycol 400, polyethylene glycol 3350, propylene glycol, a solubilizer, and an antioxidant; 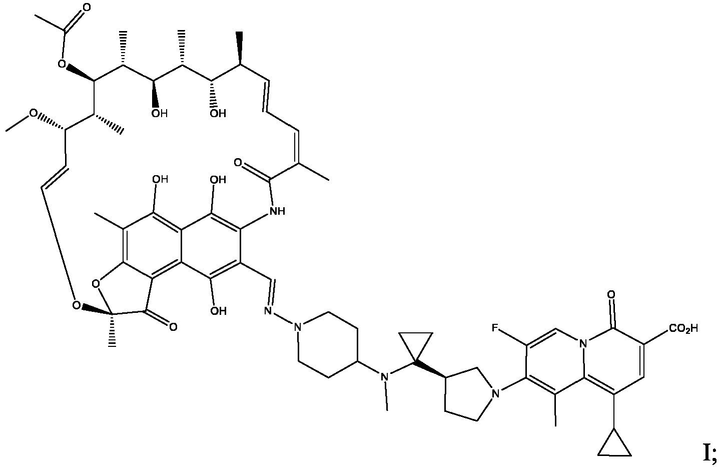
wherein based on the total mass percentage of 100%,
the content of the rifamycin-quinolizinone coupling molecule is 0.05%-0.7% by mass,
the content of polyethylene glycol 400 is 55%-65% by mass,
the content of polyethylene glycol 3350 is 25%-35% by mass,
the content of propylene glycol is 8%-12% by mass,
the content of the solubilizer is 0.1%-1% by mass, and
the content of the antioxidant is 0.3%-1% by mass.

2. The ointment of the rifamycin-quinolizinone coupling molecule according to claim 1, wherein the content of the rifamycin-quinolizinone coupling molecule is 0.3%-0.7% by mass.

3. The ointment of the rifamycin-quinolizinone coupling molecule according to claim 1, wherein the content of the rifamycin-quinolizinone coupling molecule is 0.05%-0.5% by mass.

4. The ointment of the rifamycin-quinolizinone coupling molecule according to any one of claims 1-3, wherein the content of the rifamycin-quinolizinone coupling molecule is 0.5% by mass.

5. The ointment of the rifamycin-quinolizinone coupling molecule according to any one of claims 1-4, wherein the content of polyethylene glycol 400 is 55%-60% by mass.

6. The ointment of the rifamycin-quinolizinone coupling molecule according to any one of claims 1-5, wherein the content of polyethylene glycol 400 is 56%-58% by mass.

7. The ointment of the rifamycin-quinolizinone coupling molecule according to any one of claims 1-6, wherein the content of polyethylene glycol 3350 is 28%-33% by mass.

8. The ointment of the rifamycin-quinolizinone coupling molecule according to any one of claims 1-7, wherein the content of polyethylene glycol 3350 is 30%-32% by mass.

9. The ointment of the rifamycin-quinolizinone coupling molecule according to any one of claims 1-8, wherein the content of the solubilizer is 0.3%-0.7% by mass.

10. The ointment of the rifamycin-quinolizinone coupling molecule according to any one of claims 1-9, wherein the solubilizer comprises triethanolamine.

11. The ointment of the rifamycin-quinolizinone coupling molecule according to any one of claims 1-10, wherein the content of the antioxidant is 0.5%-1% by mass.

12. The ointment of the rifamycin-quinolizinone coupling molecule according to any one of claims 1-11, wherein the antioxidant comprises one or more of vitamin C and vitamin E.

13. The ointment of the rifamycin-quinolizinone coupling molecule according to claim 12, wherein the content of vitamin C and/or vitamin E is 0.3%-0.5% by mass.

14. The ointment of the rifamycin-quinolizinone coupling molecule according to any one of claims 1-13, wherein the components of ointment of the rifamycin-quinolizinone coupling molecule comprise, based on the total mass percentage of 100%,
0.5% by mass of the rifamycin-quinolizinone coupling molecule,
56%-58% by mass of polyethylene glycol 400,
30%-32% by mass of polyethylene glycol 3350,
10% by mass of propylene glycol,
0.3%-0.7% by mass of triethanolamine,
0.3%-0.5% by mass of vitamin C by mass, and
0.3%-0.5% by mass of vitamin E.

15. A preparation method for the ointment of the rifamycin-quinolizinone coupling molecule according to any one of claims 1-14, comprising:
heating and melting polyethylene glycol 3350, propylene glycol, the solubilizer, the antioxidant, and part of polyethylene glycol 400, and mixing to obtain a first mixture,
mixing and dissolving the rifamycin-quinolizinone coupling molecule and the rest of polyethylene glycol 400 to obtain a first drug liquid, and
mixing the first drug liquid with the first mixture to obtain the ointment of the rifamycin-quinolizinone coupling molecule.

16. The preparation method according to claim 15, wherein in the step of mixing and dissolving the rifamycin-quinolizinone coupling molecule and the rest of polyethylene glycol 400 to obtain the first drug liquid, the mixing and dissolving are performed at a temperature of 50 °C.

17. The preparation method according to claim 15, wherein in the step of mixing the first drug liquid with the first mixture, the mixing is performed at a temperature of 50-53 °C.

18. A preparation method for the ointment of the rifamycin-quinolizinone coupling molecule according to any one of claims 1-14, comprising:
heating and melting polyethylene glycol 3350, propylene glycol, the solubilizer, the antioxidant, and polyethylene glycol 400, and mixing to obtain a first mixture; and
mixing the rifamycin-quinolizinone coupling molecule with the first mixture to obtain the ointment of the rifamycin-quinolizinone coupling molecule.

19. The preparation method according to claim 15 or 18, wherein the heating and melting are performed at a temperature of 70-73 °C.

20. The preparation method according to claim 18, wherein in the step of mixing the rifamycin-quinolizinone coupling molecule with the first mixture, the mixing is performed at a temperature of 50 °C.
